# EUROPEAN PATENT SPECIFICATION

(11) **EP 4 249 017 B1**
(45) Date of publication and mention of the grant of the patent: **17.12.2025**
(21) Application number: 22305377.8
(22) Date of filing: 25.03.2022
(51) Int. Cl.: A61M 5/20, A61M 5/315, A61M 5/32, A61M 5/50

(54) **AUTOMATIC INJECTION DEVICE**
AUTOMATISCHE INJEKTIONSVORRICHTUNG
DISPOSITIF D'INJECTION AUTOMATIQUE

(43) Date of publication of application: 27.09.2023
(73) Proprietor: Becton Dickinson France, 38800 Le Pont de Claix (FR)
(72) Inventor: GUIRIMAND, Anne, 38360 SASSENAGE (FR)
(74) Representative: Regimbeau

(56) References cited:
- WO-A2-2009/040672
- US-A1- 2011 218 500
- US-A1- 2018 185 583

## Description

### TECHNICAL FIELD

The present invention relates to a medical device for automatic injection of a product in a safe way, especially in emergency situations.

### TECHNICAL BACKGROUND

Some illnesses necessitate regular injections of drugs or products, for instance on a daily basis. In order to simplify the treatment, some self-injectors have been provided in order to allow the patient to perform the injection on his/her own. The patent documents US 2011/218500 A1, WO 2009/040672 A2 and US 2018/185583 A1 disclose some examples of such self-injectors.

Since the patient is usually neither a nurse nor an educated person in medical devices, such self-injectors must prove to be very simple to use and also very safe. In particular, the insertion of the needle must be performed at the right depth, the correct dose of product must be injected, that is to say a complete injection must be performed, and the injector must be deactivated after use before it is disposed of. Preferably, the needle should not be exposed, before and after use, in order to prevent any accidental needlestick injury.

Another important requirement of these self-injection devices is that they must not be able to be activated inadvertently, before the patient is ready to perform the injection, and in particular before the device is correctly applied at the right injection site.

Some automatic injection devices comprise a safety shield adapted to cover the needle tip except during injection, and a button to be pressed by the patient to trigger the injection. Before use, the button is locked in order to avoid any accidental activation. The device comprises a mechanism configured to unlock the button once the safety shield has been pushed sufficiently onto the patient's skin.

The document EP 2 921 191 discloses such an automatic injection device.

However, many automatic injection devices do not allow the user to easily understand that the injection has been completed. Some injection devices provide a visual feedback, such as the visualization of the position of the stopper at the end of the injection. Such a feedback is not sufficiently intuitive for all users.

Thus, there is a need for self-injection devices that provide an audible feedback when the dose has been completely administered to the patient.

### SUMMARY OF THE DISCLOSURE

The present invention meets this need by proposing a device for automatic injection of a product into an injection site, as defined in claim 1. Further embodiments are given in the dependent claims.

Thanks to the cooperation between the activator and the central piece, a sound is generated at the end of the injection, which can be heard by the user and thus clearly indicates that the injection has been completed. The sound may be created by the disengagement of a clipped connection, by the release of strain accumulated in the central piece and/or by a shock of the central piece onto another piece of the injection device.

In the present text, the distal end of a component or of a device is to be understood as meaning the end furthest from the user's hand and the proximal end is to be understood as meaning the end closest to the user's hand. Likewise, in the present text, the distal direction is to be understood as meaning the direction of injection, and the proximal direction is to be understood as meaning the opposite direction to the direction of injection.

In the present text, the term "axial" designates a direction parallel to the direction of injection (the direction of injection also corresponding to a longitudinal axis of the injection device) and the term "radial" designates a direction perpendicular to the direction of injection.

In preferred embodiments, taken alone or in combination if appropriate:
- the retention member of the activator comprises at least one protrusion extending radially inwardly, said protrusion engaging the proximal head so as to prevent the central piece from moving in the distal direction at least until the end of the injection;
- the proximal head of the central piece has a fork shape with two proximal legs and the proximal head is configured to disengage from the protrusion of the activator at the end of the injection, thereby generating a sound;
- the proximal head of the central piece has a fork shape with two proximal legs and the protrusion of the activator is configured to retain the central piece until after the injection and the plunger rod is configured to disengage from the distal stop of the central piece at the end of the injection, thereby generating a sound;
- the proximal head of the central piece has a harpoon shape with two distal hooks, the proximal head being in a free state in the initial position and the proximal head is configured to disengage from the protrusion of the activator at the end of the injection by constraining the hooks radially inwardly and to hit an inner wall of the plunger rod with the hooks upon release of the constraints in the hooks, thereby generating a sound;
- each flexible arm comprises an inclined inner surface tapering inwardly in the distal direction and at least one plunger retainer arranged distally from said inclined surface, the proximal flange of the plunger rod being in abutment with the at least one retainer in the initial position;
- the activator comprises at least one inner protrusion extending in the distal direction, said at least one protrusion being proximally spaced from the inclined surface of a respective flexible arm in the initial position and wherein, when the cam is in the unlocking position, said at least one inner protrusion engages the respective inclined surface to deflect the flexible arm outwardly;
- the activator comprises at least one leg extending in the distal direction, each leg comprising a tooth received in a third groove of the cam so that the rotation of the cam to the unlocking position causes the activator to translate distally to deflect the flexible arms outwardly to release the proximal flange of the plunger rod;
- the activator forms a button cooperating with the cam such that the rotation of the cam releases the button, the button thereby being able to be pressed by a user in the distal direction to unlock the plunger rod;
- the safety shield comprises at least one lug received in a respective first groove of the cam so that a movement of the safety shield in the proximal direction causes the cam to rotate;
- the first groove presents an inclined first branch and an axial second branch connected to the first branch at a proximal end, such that, in the initial position, the lug is located at the distal end of the first branch and moves towards the proximal end of the first branch when the safety shield moves from the initial position to the second position;
- the plunger rod is urged in the distal direction by a first spring;
- the safety shield is urged in the distal direction by a second spring;
- the injection device further comprises a ring supporting the medical container, the ring comprising at least one finger projecting radially outwardly, the cam further comprising at least one second groove receiving the respective at least one finger, the at least one second groove comprising a proximal portion, a distal portion and an inclined portion connecting the proximal portion to the distal portion, wherein the ring is locked at the proximal portion of the second groove and moves along inclined portion to the distal portion when the plunger rod pushes the medical container from the initial position to the injection position;
- the at least one finger engages an axial groove of the body.

### BRIEF DESCRIPTION OF THE DRAWINGS

Further features and advantages of the invention will be disclosed in the following detailed description, based on the appended drawings, wherein:
- FIG. 1 is a perspective view of the body and the cap of the injection device;
- FIG. 2 is an exploded view of the body and the cap;
- FIG. 3 is an exploded view of the medical container and the needle shield of the injection device;
- FIG. 4 is an exploded view of the plunger rod and the medical container in one embodiment;
- FIG. 5 is an exploded view of the plunger rod and the central piece of FIG. 4;
- FIG. 6 is a perspective view of the cap and the needle shield;
- FIG. 7 is a perspective view of the medical container and the supporting ring;
- FIG. 8 is a perspective view of the safety shield;
- FIG. 9 is a perspective view of the button;
- FIG. 10 is a perspective view of the cam;
- FIG. 11 is a perspective view of the assembly of the safety shield, the cam and the ring;

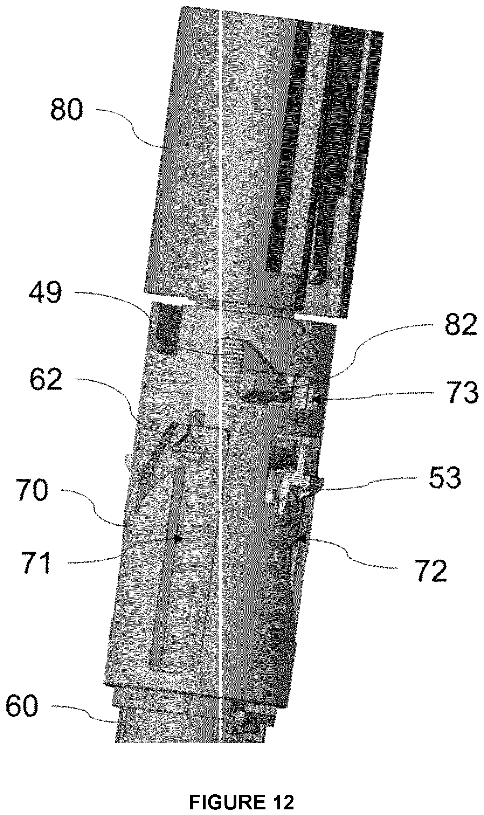

- FIG. 12 is a perspective view of the assembly of the safety shield, the cam, the ring and the activator;
- FIGS. 13 and 14 are sectional views of the upper part of the injection device in the initial position, with the plunger rod and the central piece of FIG. 4;
- FIG. 15 is a sectional view of the injection device with the plunger rod and the central piece of FIG. 4 at the end of the injection;
- FIG. 16A and 16B are alternative enlarged views of the proximal end and the distal end of the central piece at the end of the injection in the embodiment of FIG. 15;
- FIG. 17 is an exploded view of the plunger rod and the medical container in another embodiment;
- FIG. 18 is an exploded view of the plunger rod and the central piece of FIG. 17;
- FIGS. 19 and 20 are sectional views of the upper part of the injection device in the initial position, with the plunger rod and the central piece of FIG. 17;
- FIG. 21 is a sectional view of the injection device with the plunger rod and the central piece of FIG. 17 at the end of the injection;
- FIG. 22A and 22B are enlarged views of the proximal end and the distal end of the central piece at the end of the injection in the embodiment of FIG. 15.

### DETAILED DESCRIPTION OF EMBODIMENTS

Unless otherwise specified, the components of the injection device are represented in their initial position, before use of the injection device. However, as will be explained below, some of the components are movable in translation and/or in rotation relative to each other to perform the injection. Thus, the description will refer not only to the initial position of the components, but also to one or more operative positions that are reached during the injection process.

In a first embodiment, not covered by the claimed invention, the injection device requires three actions from the user to trigger the injection:
(1) removing a cap;
(2) pushing firmly the device onto the patient's skin to unlock a button; and
(3) pressing the unlocked button to activate the device and trigger injection.

In such a device, in the initial position, exposure of the needle and injection of the product are prevented by the fact that the needle and the medical container containing the product for injection are housed within the body of the injection device and that the plunger rod is locked in translation in the body. To perform an injection, a safety shield is applied onto the patient's skin at an injection site. The safety shield is thus caused to translate in the proximal direction, which causes a cam coupled to the safety shield to rotate until an activation position. In said activation position, the cam unlocks the button. Said button can then be pressed by the user, which results in unlocking the plunger rod and allowing it to push the medical container in the distal direction to an injection position and push the stopper to expel the product from the medical container into the patient's body.

In a second embodiment, the injection device requires only two actions from the user to trigger the injection, which are:
(1) removing the cap; and
(2) firmly pushing the device onto the patient's skin.

In such a device, in the initial position, exposure of the needle and injection of the product are prevented by the fact that the needle and the medical container containing the product for injection are housed within the body of the injection device and that the plunger rod is locked in translation in the body. To perform an injection, a safety shield is applied onto the patient's skin at an injection site. The safety shield is thus caused to translate in the proximal direction, which causes a cam coupled to the safety shield to rotate until unlocking an activator. Said unlocking of the activator triggers a continuous injection phase in which a plunger rod is unlocked and pushes a stopper in the distal direction. As a result, a medical container engaging the stopper is caused to translate in the distal direction to the injection position - thereby allowing the needle to pierce the patient's skin at the right injection depth. Further, the movement of the stopper in the medical container in the distal direction expels the product from the medical container into the patient's body.

In both embodiments, the plunger rod is coupled to a central piece which is configured to hit another component of the injection device at the end of the injection or to otherwise produce a sound.

In the following description, only injection devices according to the second embodiment have been illustrated. However, the disclosure also applies in a similar way to injection devices according to the first embodiment. Indeed, the first and second embodiments mainly differ by the fact that, in the second embodiment, the activator is pulled by the cam without any additional action from the user to trigger the injection, whereas, in the first embodiment, the activator is a button that has to be pressed by the user after the activation position of the cam has been reached to trigger the injection, but the architecture of the activator and the way it cooperates with the central piece of the plunger rod allows in both case the arrangement of the plunger rod and the central piece that will be described below to generate a sound at the end of the injection.

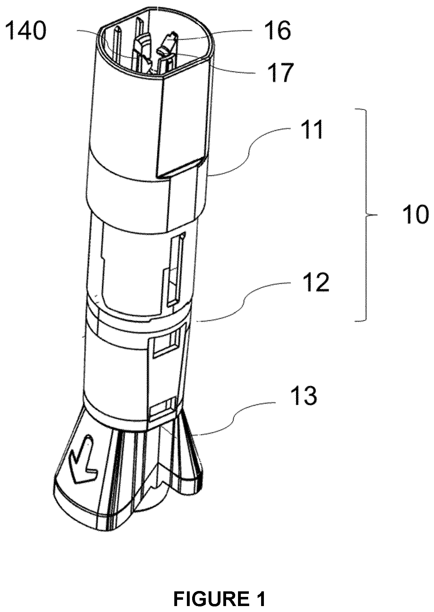

FIG. 1 shows a perspective view of a device for automatic injection according to an embodiment of the present disclosure.

The device comprises a housing 10 comprised of an upper body 11 and a lower body 12 that may be rigidly connected to each other.

The housing 10 has an outer shape adapted to be held in a user's hand. In general, the outer surface of the housing 10 is intended to be gripped by the palm and the fingers of the user's hand.

The connexion of the upper and lower bodies can be a snap-fit connection, screw-type connection, bayonet connection, or other means of connecting two parts together, in an unreleasable way or not. When the device is of a single use type, the means for connecting the upper body to the lower body are made unreachable to the user.

A cap 13 is removably connected to a distal end of the housing 10. The cap 13 can be connected to the lower body 12 by a snap-fit connection or by any other type of connection allowing removal of the cap 13 by an axial movement in the distal direction.

A medical container 20 such as, for example, a syringe, is received in at least one of the upper and lower bodies 11, 12.

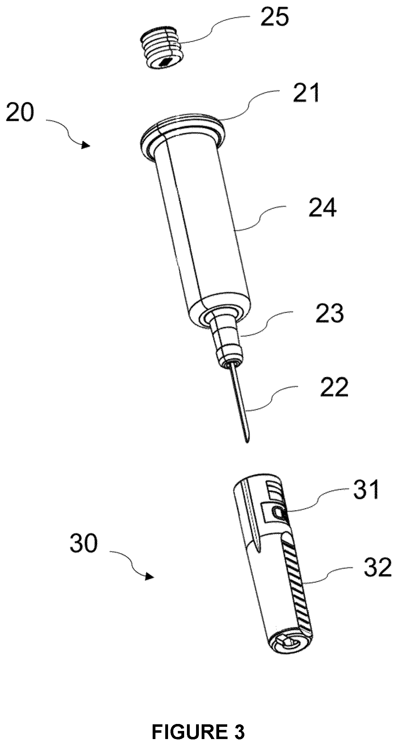

As shown in FIG. 3, the medical container 20 has a radial flange 21 defined at an open proximal end, and an injection needle 22 at a substantially closed distal end 23. Lateral walls extend between the proximal and distal ends and define a barrel 24 sized and shaped to contain a predetermined amount of a product for injection. The injection needle 22 may be fixed to the distal end 23, or removable therefrom, as a matter of design choice. The injection needle 22 is in fluid communication with the barrel 24 and provides an outlet port of the medical container 20 for the product.

A needle shield 30 is provided at the distal end of the medical container 20 to cover and protect the needle 22 before use of the device 1. The needle shield 30 also provides for a sealing means of the distal end 23 of the medical container before use. To that end, the needle shield 30 may comprise an inner elastomeric shield 31 sealingly engaging the distal end of the medical container and an outer rigid cap 32 surrounding the inner shield.

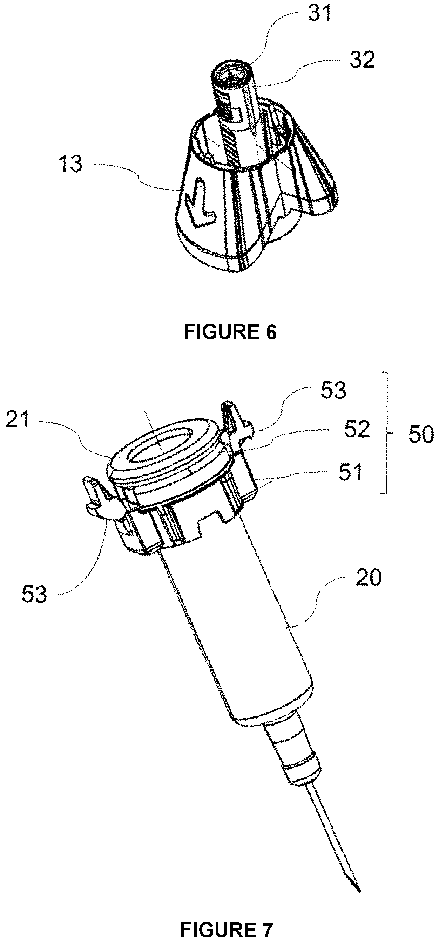

Before use (see FIG. 6), the cap 13 engages the outer rigid cap 32 so that removal of the cap 13 simultaneously removes the inner elastomeric shield 31 and the outer rigid cap 32 from the distal tip of the medical container.

A stopper 25 is provided in the container 20 and is slidingly movable within the barrel 24. Movement of the stopper in the distal direction causes the product to be expelled from said medical container 20 through the injection needle 22 during the injection of the product into the patient. The stopper is typically made of an elastomeric material. The inner surface of the barrel and/or the outer surface of the stopper may be lubricated in order to reduce the gliding force of the stopper within the barrel.

A plunger rod 40 for causing the stopper to move with respect to the medical container 20, as will be explained later, is received within the proximal portion of the upper body 11.

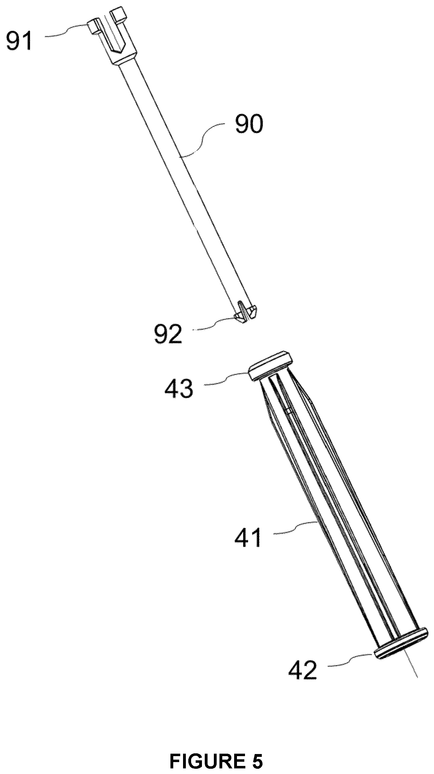

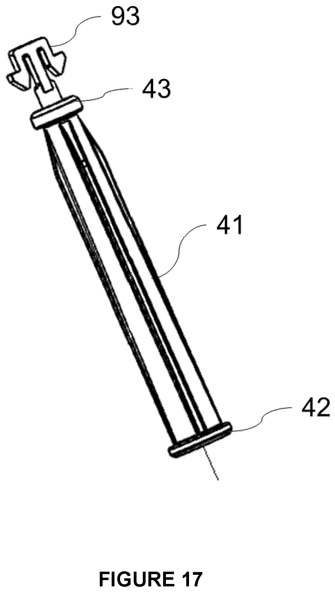

As shown in FIGS. 5 and 17, the plunger rod 40 comprises a hollow shaft 41 provided with a distal flange 42 and a proximal flange 43. The shaft 41 may comprise one or more axial rib(s) designed for increasing its stiffness. The shaft 41 comprises an inner cavity open at both ends, the proximal portion having a smaller diameter than the distal portion, said portions being separated by a radial wall forming an axial abutment 45.

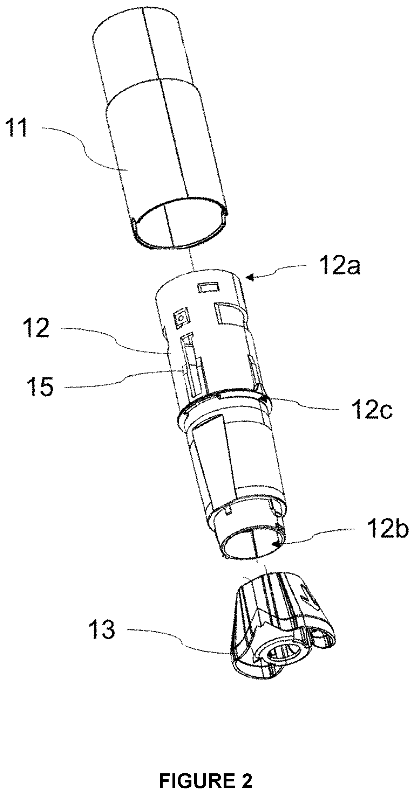

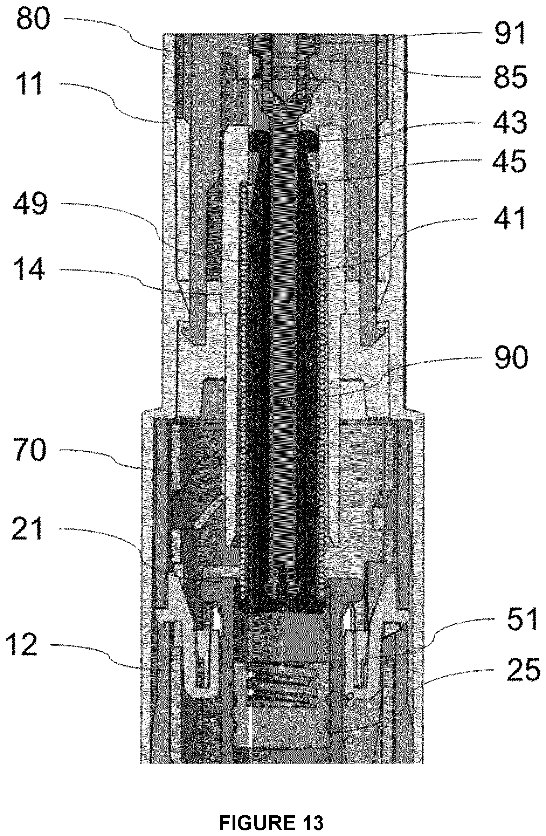

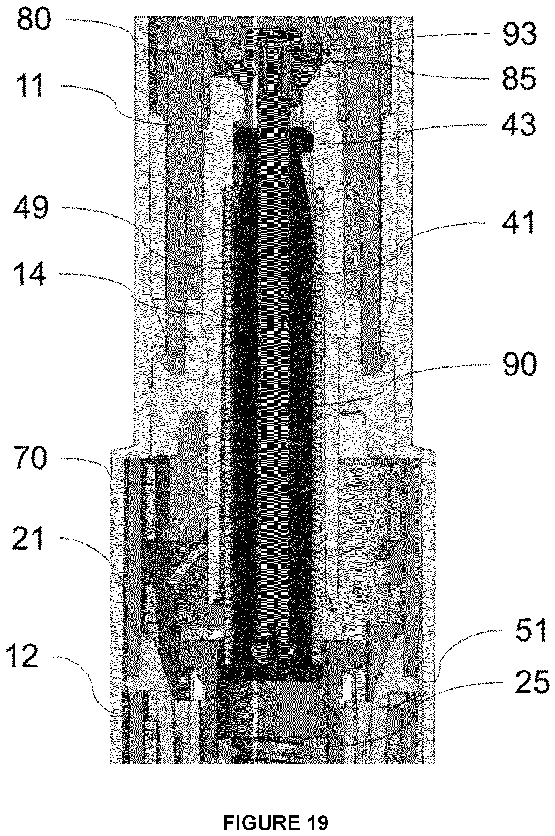

Referring to FIGS. 1 and 2, the upper body 11 has a generally cylindrical shape and is open at both ends. The distal end is connected to the lower body 12. The upper body further comprises an inner cylinder 14 receiving the plunger rod 40. The inner cylinder 14 comprises an axial wall parallel to the outer wall of the upper body and connected to said outer wall by a radial wall. The inner cylinder 14 has an open distal end and a proximal end comprising at least one flexible arm 140 comprising an inclined inner surface 16 tapering inwardly in the distal direction and at least one plunger retainer 17 arranged distally from said inclined surface 16. Preferably, the proximal end comprises two flexible arms diametrically opposite to each other, or even two pairs of diametrically opposite flexible arms, with adjacent arms spaced by a 90° angle. For example, each plunger retainer 17 is in the form of a clip extending radially inwardly, comprising a proximal surface perpendicular to the direction of injection engaging the distal surface of the proximal flange 43 of the plunger rod in the initial position. Thus, in the initial position, the plunger rod 40 is prevented from moving distally by the plunger retainer(s) 17 (see FIG. 13 and 19). As will be explained in more detail below, the flexible arms are designed to be able to deflect outwardly when a force is exerted onto the inclined surface 16 in the distal direction.

A central piece 90 is slidably arranged within the hollow shaft 41 of the plunger rod. The piece 90 comprises a proximal head 91 or 93 and a distal stop 92.

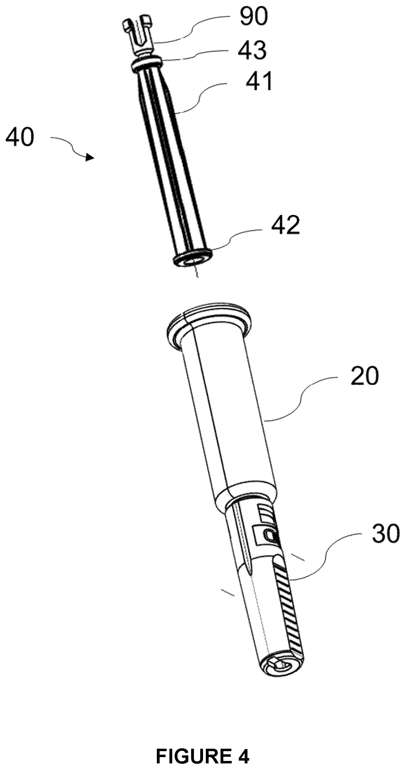

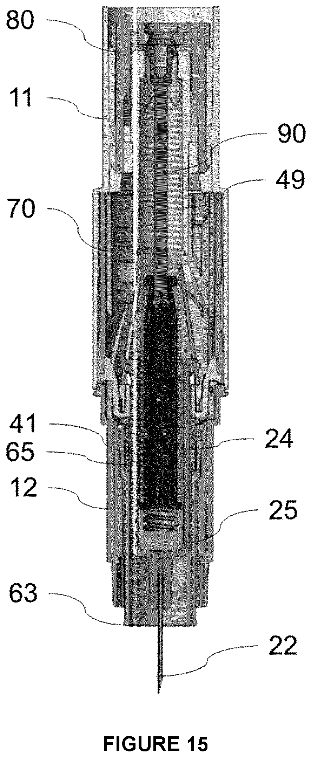

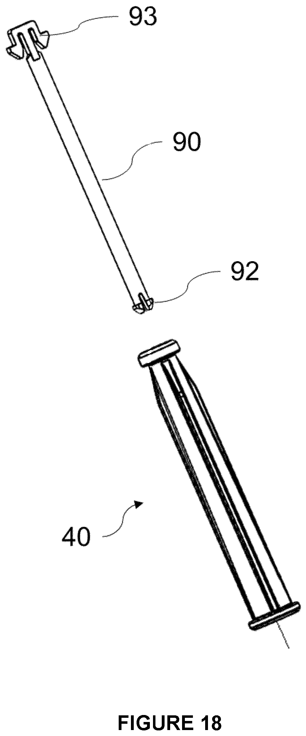

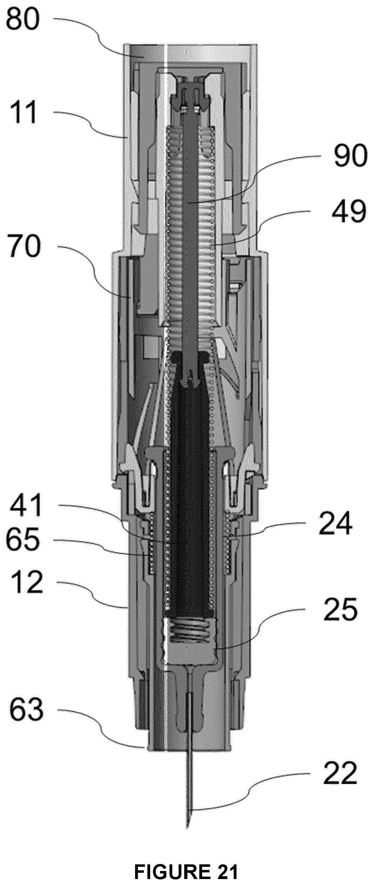

In some embodiments (see FIGS. 4, 5 and 13 to 16B), the proximal head 91 has a fork shape with two proximal legs provided with a respective tooth extending radially outwardly. In other embodiments (see FIGS. 17 to 22B), the proximal head 93 has a harpoon shape with two distal flexible hooks.

The distal stop 92 has a smaller diameter than the distal portion of the cavity of the plunger rod, but a greater diameter than the proximal portion of said cavity.

In the initial position, the head 91 or 93 of the central piece 90 protrudes proximally from the proximal flange 43 of the plunger rod and the distal stop 92 extends within the hollow shaft 41.

An activator 80 is slidably mounted in the proximal portion of the upper body 11. The activator substantially surrounds the proximal end of the inner cylinder 14.

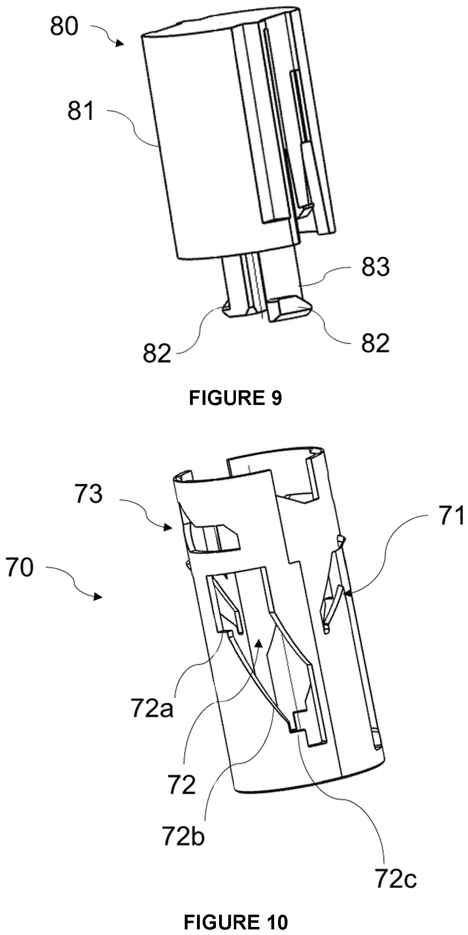

As shown in FIG. 9, the activator 80 comprises an axial skirt 81 having a generally cylindrical shape open at its distal end and closed at its proximal end. The activator comprises at least one leg 83 extending distally from the skirt 81. Preferably, the activator comprises two legs 83 diametrically opposite to each other. The distal end of each leg is provided with a tooth 82 extending radially outwardly.

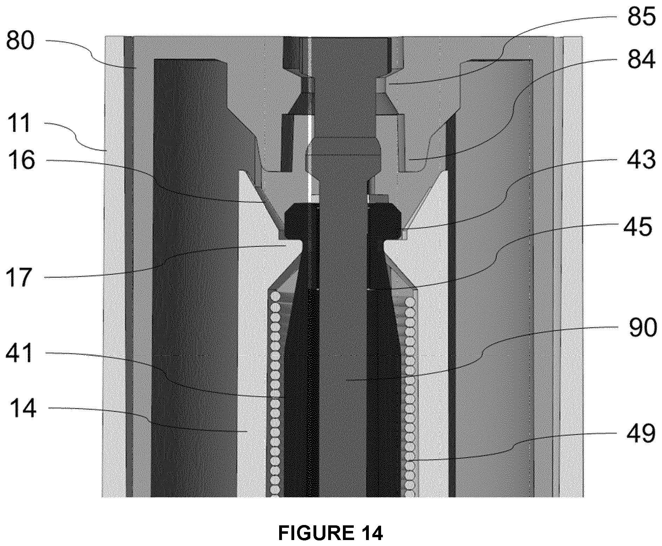

As best seen in FIG. 14, the proximal end of the activator comprises at least one inner protrusion 84 extending in the distal direction towards the inner cylinder 14. In some embodiments, said protrusion may form a continuous annular rib. In other embodiments, at least two separate protrusions 84 are provided, each angularly aligned with a flexible arm 140. In the initial position, said at least one protrusion 84 is proximally spaced from the inclined surface 16 of each flexible arm.

As also shown in FIGS. 13 and 14, the proximal end of the activator further comprises at least one inner protrusion 85 extending radially inwardly and cooperating with the teeth of the head 91 of the central piece 90 to lock the central piece in translation in the distal direction. In some embodiments, said protrusion may form a continuous annular rib. In other embodiments, at least two separate protrusions 85 are provided, each angularly aligned with a tooth of the head 91 of the central piece.

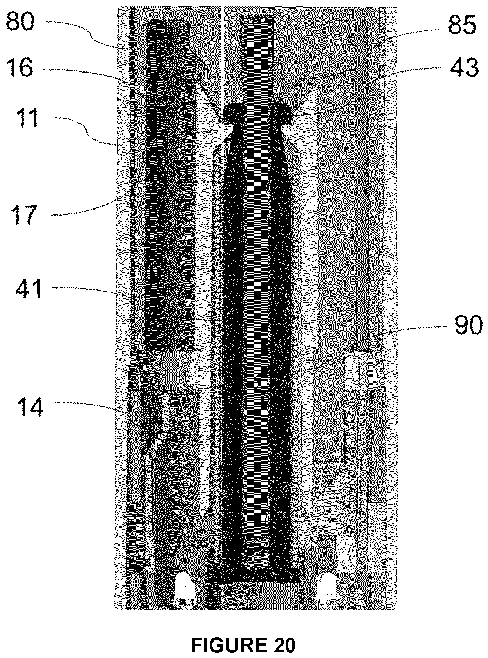

When the central piece has a harpoon-shaped proximal end 93, the hooks are in a free (non-constrained) state in the initial position (see FIGS. 19 and 20). In such a way, creep of the hooks, which may hinder the generation of the audible sound, can be avoided.

A first spring 49 is arranged around the shaft 41 of the plunger rod, the distal end of the spring 49 bearing onto the distal flange 42 and the proximal end of the spring 49 bearing onto an inner surface of the proximal end of the inner cylinder 14. In the initial position (see FIGS. 13 and 19), the spring is in a compressed state. As a result, the spring 49 urges the plunger rod 40 in the distal direction. However, as noted above, in the initial position, the plunger rod is locked by the plunger retainers 17 and thus is prevented from translating in the distal direction.

As illustrated in FIG. 7, the medical container 20 is mounted in a supporting ring 50. The ring may comprise a distal rigid portion 51 and a proximal elastomeric portion 52. The barrel of the medical container is inserted into the supporting ring 50 so that the proximal flange 21 bears onto the proximal surface of the proximal elastomeric portion 52. The medical container may be maintained in a fixed position relative to the ring by frictional engagement between the elastomeric portion 52 and the barrel.

The ring 50 comprises at least one finger 53 protruding radially from the rigid portion 51. Preferably, the ring comprises two fingers 53 diametrically opposite to each other.

The lower body forms a housing which receives at least partially the medical container 20 and the ring 50. As will appear later, the medical container 20 is movable relative to the lower body between an initial position, in which the tip of the needle does not extend beyond the distal end of the lower body, and an injection position, distally spaced relative to said initial position and in which the tip of the needle extends beyond the distal end of the lower body and is exposed over a predetermined length.

The lower body has a general cylindrical shape and is open at both ends. As shown in FIG. 2, the lower body 12 has a distal part 12b and a proximal part 12a, the diameter of the proximal part 12a being greater than the diameter of the distal part 12b. The proximal part 12a and the distal part 12b are joined by a radial wall 12c. The proximal surface of the radial wall 12c forms an abutment surface for the ring 50.

The lower body comprises at least one axial groove 15 receiving a respective finger 53 of the ring 50. Preferably, the lower body has two axial grooves 15 diametrically opposite to each other, and the ring has two fingers, each received in a respective axial groove. As will be explained in more detail below, such axial groove serves as a guide for axial movement of the ring (and the container) relative to the lower body. Advantageously, said groove is located in a proximal portion of the lower body, which is covered by the upper body; thus, the groove 15 and the finger 53 are not accessible by a user from the outside of the injection device.

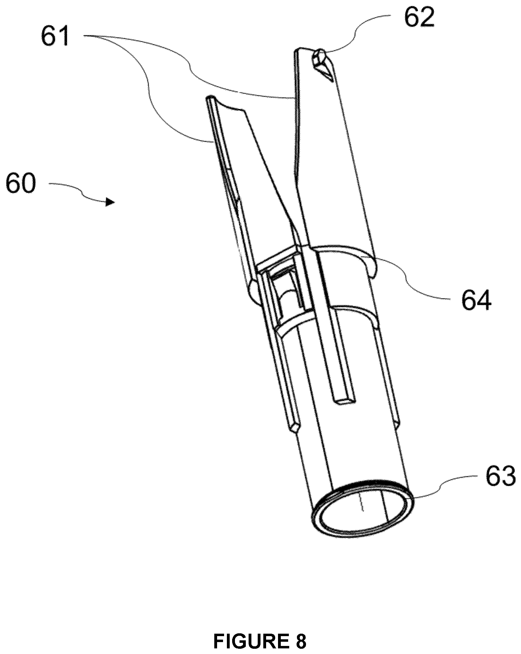

The injection device also includes a safety shield 60 that is at least partially received within a distal portion of the lower body. As shown in FIG. 8, the safety shield 60 comprises two opposite tongues 61 extending proximally. Each tongue 61 comprises a lug 62 extending radially outwardly.

The distal end of the safety shield 60 is advantageously provided with a radial flange 63 forming a bearing surface for applying the injection device onto the patient's skin, the width of the flange being chosen so as to distribute the pushing force on a sufficiently large surface for not injuring the patient.

The safety shield is coupled to a cam 70 pivotably mounted in the lower body 12.

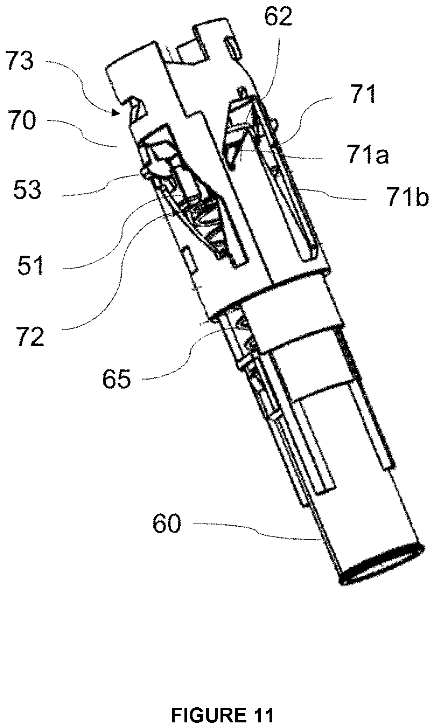

As shown in FIGS. 10 and 11, the cam 70 has a generally cylindrical shape and is open at both ends.

The cam 70 presents at least one first groove 71, preferably two first grooves diametrically opposite to each other.

Each first groove 71 comprises an inclined (i.e. non-axial) first branch 71a and an axial second branch 71b connected to the first branch at a proximal end of both branches 71a, 71b. Otherwise said, each first groove has the shape of number "1".

Each lug 62 of the safety shield slidingly engages a respective first groove 71 in the cam 70.

In the initial position, the lug 62 is located at the distal end of the first branch 71a and moves towards the proximal end of the first branch 71a when the safety shield 60 moves from the initial position to the injection position. As a result, such a movement of the safety shield 60 in the proximal direction causes the cam to rotate by an angle depending on the slope of the first branch 71a and the stroke of the lug 62.

When the lug 62 reaches the proximal end of the first branch 71a and engages the second branch 71b, the activator is unlocked, as will be explained below.

A second spring 65 is arranged between the proximal end of the rigid portion of the ring 50 and the flange 64 of the safety shield. In the initial position, the second spring 65 may be in a relaxed state, but a movement of the safety shield 60 in the proximal direction compresses the second spring 65. As a result, if the user releases the pressure applied onto the injection device, the second spring 65 urges the safety shield 60 in the distal direction.

The cam 70 further comprises at least one second groove 72 (preferably two second grooves 72 diametrically opposite to each other). Each second groove 72 comprises a proximal portion 72a extending perpendicular to the direction of injection and a distal portion 72c parallel to the proximal portion, and an inclined (i.e. non-axial) portion 72b connecting the proximal portion 72a to the distal portion 72c. Otherwise said, the proximal and distal portions 72a, 72c of each second groove are spaced both in the axially and angularly. The axial distance between the proximal portion 72a and the distal portion 72c of the second groove 72 is equal to the penetration depth of the needle within the patient's body.

The ring 50 is at least partially received within the cam with each finger 53 of the ring being in sliding engagement within a respective second groove 72.

In the initial position, each finger 53 of the ring is in the proximal portion 72a of the respective second groove 72.

The cam 70 further comprises at least one third groove 73 (preferably two second grooves 73 diametrically opposite to each other). Each third groove 73 comprises an inclined (i.e. non-axial) portion 73a and a proximal portion 73b extending perpendicular to the direction of injection connected to the inclined portion 73a. Each tooth 82 of the activator 80 is slidingly received in a respective third groove 73. In the initial position, the tooth 82 is located at the proximal end of the inclined portion 73a.

When the cam 70 is caused to pivot by the movement of the safety shield 60 in the proximal direction along the first branch 71a of the first groove of the cam (as explained above), the tooth 82 of the activator 80 slides along the inclined portion 73a of the third groove 73, which generates a translation of the activator 80 in the distal direction, until the tooth 82 reaches the distal portion 73b of the third groove 73. The first and third grooves 71, 73 are designed such that the tooth 82 reaches the distal portion 73b of the third groove 73 (which corresponds to the unlocking position of the cam) when the lug 62 arrives at the connection between the first and second branches 71a, 71b of the first groove 71 (which corresponds to the second position of the safety shield). However, said rotation of the cam 70 does not cause any movement of the ring 50.

The functioning of the injection device 1 will now be explained.

The injection device is provided to a user ready-to-use, with the cap closing the distal end of the body.

The medical container is filled with a predetermined dose of an injectable product - preferably a single dose thus providing a one-time use or disposable injection device.

Prior to use, the user removes the cap and the needle shield, without rotation of said needle shield. The injection device is thus in its initial position.

The user then places the safety shield against the patient's skin at an injection site. The patient may be the user or another person.

As the device is pressed against the patient's skin, the safety shield is caused to move in the proximal direction and into the lower body.

Due to the above-described safety features, the user cannot activate the device (i.e., cause the container to move from its initial position to its injection position and cause the plunger to push the stopper within the barrel) until the safety shield is caused to move a predetermined distance in the proximal direction so as to allow the cam to unlock the activator.

When the device is pressed against the patient's skin (and the safety shield is moved out of its initial position in the proximal direction until the activator is unlocked, the device is automatically activated to begin an injection.

This unlocking of the activator causes the plunger rod to be unlocked and to push the stopper in the distal direction. Said movement of the stopper thus causes a movement of the whole medical container in the distal direction from its initial position to its injection position, which also causes the needle to pierce the patient's skin.

Then, the plunger rod is still pushing the stopper in the distal direction in the barrel, which causes the injectable product to automatically be expelled from the container and into the patient's skin by moving the plunger rod in the distal direction so as to push the stopper.

It is to be noted that, in said second embodiment, once the activator has been unlocked, the unlocking of the plunger rod, the movement of the stopper and of the medical container happen during a continuous injection phase, without any further action from the user. In addition, the second position of the safety shield and the unlocking position of the cam are not stable positions but merely intermediate (temporary) positions reached during the triggering of the injection device.

By contrast, in the first embodiment, once the button formed by the activator has been unlocked, the user has to press the button in the distal direction to release the plunger rod and thereby trigger the injection.

Once the injection is complete, the user removes the device from the injection site and the safety shield is caused to automatically extend from the lower body to cover the now-contaminated tip of the needle. Advantageously, even if the user removes the device from the injection site before the injection is complete, the safety shield will automatically extend over the tip of the needle. Once the injection device is removed from the injection site and the safety shield is extended over the tip of the needle, the shield is locked in place and cannot thereafter be moved from its locked position in the proximal direction to expose the tip of the needle. The used injection device is thus rendered safe for handling and disposal.

Referring to the figures, when the user applies the injection device on the injection site by means of the bearing surface 63 of the safety shield 60, a distal force is exerted on the lower body 12 thereby causing the safety shield 60 to move relative to said lower body from the initial position, to a second position in which the cam unlocks the activator.

During this proximal translation of the safety shield 60 to its second position, each lug 62 translates along the inclined branch 71a of the cam 70, thereby causing the cam 70 to rotate until the connection with the axial branch 71b, which corresponds to the unlocking position of the cam.

If the user releases the pushing force before reaching the second position, the compressed second spring 65 causes the safety shield to move back to the distal position in order to protect the needle.

The rotation of the cam pulls the activator 80 in the distal direction so that, in the unlocking position, the tooth 82 of the activator reaches the distal portion 73b of the third groove 73.

In said unlocking position, the inner protrusion 84 of the activator engages the inclined surface 16, which causes each flexible arm of the inner cylinder 14 to deflect outwardly. Thus, the plunger retainers 17 disengage from the proximal flange 43 of the plunger rod.

As a result, the plunger rod 40 is caused to move in the distal direction under the distal force of the spring 49.

The distal end 42 of the plunger rod 40 engages the stopper and pushes it in the distal direction. The stopper which is in frictional engagement with the barrel thus causes the whole medical container to move in the distal direction.

Since the medical container is supported by the ring 50, the ring 50 is caused to translate in the distal direction with the medical container. During said translation, the fingers 53 of the ring 50 move within the second groove 72 of the cam. More precisely, each finger 53 engages the inclined portion 72b of the respective second groove 72, until the finger 53 reaches the distal portion 72c of the second groove, which prevents any further movement of the ring 50 and the medical container in the distal direction. Said translation of the ring 50 causes the cam 70 to further rotate, which allows the lug 62 to engage the second branch 71b of the first groove 71.

The medical container has thus reached its injection position. In this position, the needle protrudes from the distal end of the lower body 12 and pierces the patient's skin. As noted above, the axial stroke of the finger 53 between the proximal and distal portions 72a, 72c of the second groove 72 of the cam 70 defines the penetration depth of the needle. This ensures that the product be injected at the right depth between the patient's body.

The plunger rod continues pushing the stopper to expel the product from the barrel through the needle until the stopper reaches the distal end of the barrel.

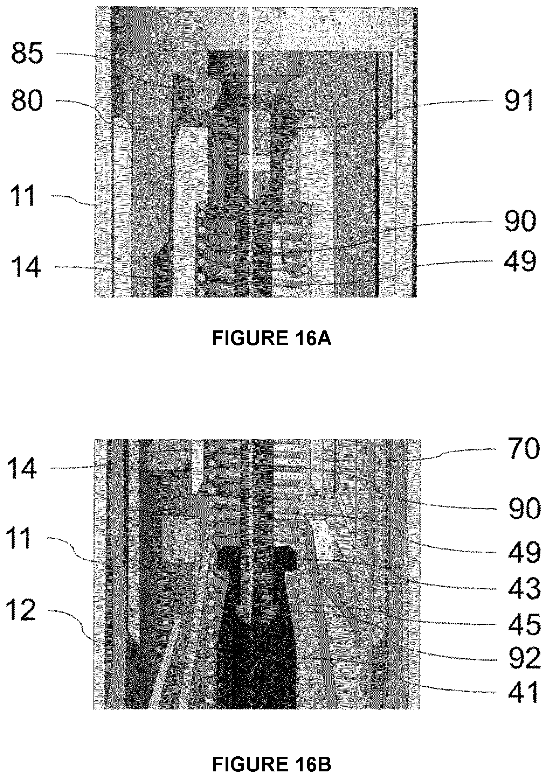

During almost all the duration of the injection phase, the head 91 of the central piece remains retained by the protrusions 85 of the activator 80. Thus, the plunger rod 40 slides distally along the central piece 90, until the stop 92 abuts the abutment 45 inside the hollow shaft 41. At this stage, since the injection is not yet completed, the plunger rod 40 which is still urged in the distal direction by the spring 49 pulls the central piece 90 and causes the head 91 of the central piece to get out of the protrusions 85 of the activator 80 (see FIG. 16A), which generates an audible sound. Alternatively, the head 91 of the central piece may remain fixed to the activator 80 thanks to the protrusions 85 but the stop 92 of the central piece gets out of the proximal end of the plunger rod which is still urged in the distal direction by the spring 49 (see FIG. 16B), thereby also generating an audible sound. The skilled person is able to design the shape and dimensions of the protrusions 85, the head 91, the stop 92 and the abutment 45 to impart an adapted stiffness or flexibility of the interacting components depending on the desired situation generating the audible sound.

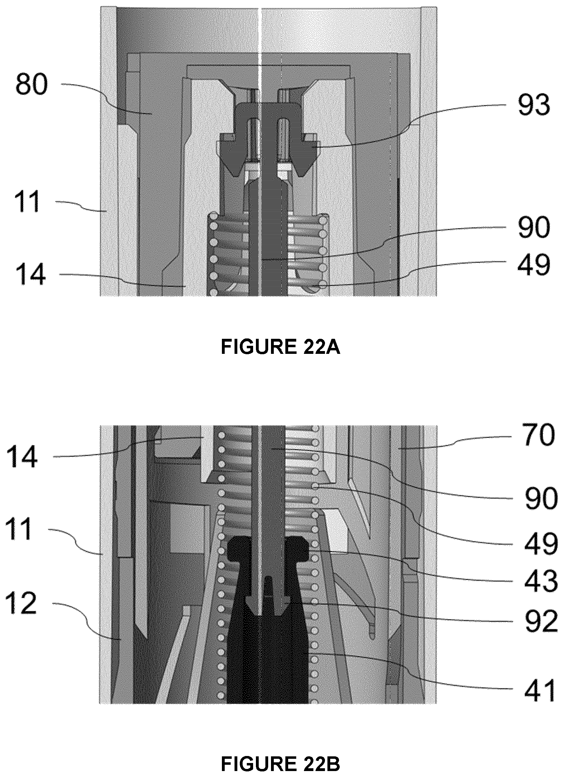

When the central piece has a harpoon-shaped proximal head 93, during almost all the duration of the injection step, the head 93 of the central piece remains retained by the protrusions 85 of the activator 80. Thus, the plunger rod 40 slides distally along the central piece 90, until the stop 92 abuts the abutment 45 inside the hollow shaft 41. At this stage, since the injection is not yet completed, the plunger rod 40 which is still urged in the distal direction by the spring 49 pulls the central piece 90 and causes the head 93 of the central piece to get out of the protrusions 85 of the activator 80. As a result, the hooks are first strained toward each other for passing through the protrusions 85 and then return to their unstrained position (see FIGS. 22A and 22B). Thus, in this case, the sound is produced by the release of the strain accumulated in the head further to the disengagement of the head 93 from the activator 80.

Once the injection is completed, the user withdraws the injection device from the patient's skin.

This withdrawal causes the second spring 65 to push the safety shield 60 in the distal direction, the lugs 62 sliding within the second branch 71b of the first groove 71 of the cam 70, until a final position in which the safety shield covers and protects the needle. When in said final position, the safety shield is locked against proximal movement thereby preventing unintended access to the contaminated needle.

The injection device is thus very easy to use, even in emergency situations, and provides an audible feedback that allows a user to easily understand that the injection has been completed. In addition, the injection device is very safe since it prevents accidental needlestick injuries even in case said device is removed from the injection site before the injection of the product is actually completed.

## Claims

1. Automatic injection device (1) comprising:
- a body (10);
- a medical container (20) comprising a barrel (24) containing a product for injection, a distal tip (23) provided with an injection needle (22) and a stopper (25) in sliding engagement within the barrel, the medical container being slidably arranged in the body (10) between a proximal position and a distal position;
- a safety shield (60) slidably mounted within the body (10) between a distal initial position and a proximal second position;
- a plunger rod (40) slidably arranged in the body (10), comprising a hollow shaft (41) including an inner axial abutment (45) and a distal flange (42) adapted to push the stopper (25) in the distal direction, the plunger rod comprising a proximal flange (43) releasably locked in the initial position by at least one flexible arm (140) of the body;
- a cam (70) cooperating with the safety shield (60) so that a translation of the safety shield (60) from the distal initial position to the proximal second position causes the cam (70) to rotate within the body (10) to an unlocking position;
- an activator (80) slidably mounted at a proximal end of the body (10) and comprising at least one leg (83) extending in the distal direction, each leg (83) comprising a tooth (82) received in a third groove (73) of the cam (70), so that the rotation of the cam (70) to an unlocking position causes the activator (80) to translate distally to deflect the flexible arms (140) outwardly to release the proximal flange (43) of the plunger rod, the activator (80) being movable between an initial position in which the activator is proximally spaced from the flexible arms (140) and an unlocking position in which the activator causes the flexible arms (140) to deflect outwardly to release the proximal flange (43) of the plunger rod, thereby allowing the plunger rod to move in the distal direction; and
- a central piece (90) comprising a proximal head (91, 93) and a distal stop (92), the central piece (90) extending in the hollow shaft (41) of the plunger rod such that the proximal head is locked by a retention member (85) of the activator proximally from the proximal flange (43) of the plunger rod in the initial position, so that, when the plunger rod moves in the distal direction, the plunger rod slides distally along the central piece (90) until the stop (92) engages the abutment (45),
wherein the retention member (85), the stop (92), the proximal head (91, 93) and the abutment (45) are designed to allow disengaging either the central piece (90) from the activator (80) or the plunger rod (40) from the central piece (90) at the end of the injection such that said disengagement results in the generation of a sound.

2. Automatic injection device according to claim 1, wherein the retention member (85) of the activator comprises at least one protrusion extending radially inwardly, said protrusion engaging the proximal head (91, 93) so as to prevent the central piece (90) from moving in the distal direction at least until the end of the injection.

3. Automatic injection device according to claim 2, wherein the proximal head (91) of the central piece has a fork shape with two proximal legs and the proximal head is configured to disengage from the protrusion (85) of the activator at the end of the injection, thereby generating a sound.

4. Automatic injection device according to claim 2, wherein the proximal head (91) of the central piece has a fork shape with two proximal legs and the protrusion of the activator is configured to retain the central piece (90) until after the injection and the plunger rod (40) is configured to disengage from the distal stop (92) of the central piece (90) at the end of the injection, thereby generating a sound.

5. Automatic injection device according to claim 2, wherein the proximal head (93) of the central piece has a harpoon shape with two distal hooks, the proximal head being in a free state in the initial position and the proximal head is configured to disengage from the protrusion (85) of the activator at the end of the injection by constraining the hooks radially inwardly then releasing the constraints in the hooks, thereby generating a sound.

6. Automatic injection device according to any one of claims 1 to 5, wherein each flexible arm (140) comprises an inclined inner surface (16) tapering inwardly in the distal direction and at least one plunger retainer (17) arranged distally from said inclined surface (16), the proximal flange (43) of the plunger rod being in abutment with the at least one retainer (17) in the initial position.

7. Automatic injection device according to claim 6, wherein the activator (80) comprises at least one inner protrusion (84) extending in the distal direction, said at least one protrusion (84) being proximally spaced from the inclined surface (16) of a respective flexible arm (140) in the initial position and wherein, when the cam (70) is in the unlocking position, said at least one inner protrusion engages the respective inclined surface (16) to deflect the flexible arm (140) outwardly.

8. Automatic injection device according to any one of claims 1 to 7, wherein the safety shield (60) comprises at least one lug (62) received in a respective first groove (71) of the cam (70) so that a movement of the safety shield (60) in the proximal direction causes the cam to rotate.

9. Automatic injection device according to claim 8, wherein the first groove (71) presents an inclined first branch (71a) and an axial second branch (71b) connected to the first branch at a proximal end, such that, in the initial position, the lug (62) is located at the distal end of the first branch (71a) and moves towards the proximal end of the first branch when the safety shield (60) moves from the initial position to the second position.

10. Automatic injection device according to any one of claims 1 to 9, wherein the plunger rod (40) is urged in the distal direction by a first spring (49).

11. Automatic injection device according to any one of claims 1 to 10, wherein the safety shield (60) is urged in the distal direction by a second spring (65).

12. Automatic injection device according to any one of claims 1 to 11, further comprising a ring (50) supporting the medical container (20), the ring comprising at least one finger (53) projecting radially outwardly, the cam (70) further comprising at least one second groove (72) receiving the respective at least one finger (53), the at least one second groove (72) comprising a proximal portion (72a), a distal portion (72c) and an inclined portion (72b) connecting the proximal portion (72a) to the distal portion (72c), wherein the ring (50) is locked at the proximal portion (72a) of the second groove (72) and moves along inclined portion (72c) to the distal portion (72b) when the plunger rod pushes the medical container from the initial position to the injection position.

13. Automatic injection device according to claim 12, wherein the at least one finger (53) engages an axial groove (15) of the body.

## Patentansprüche

1. Automatische Injektionsvorrichtung (1), umfassend:
- einen Körper (10);
- einen medizinischen Behälter (20), der einen Zylinder (24), der ein Produkt zur Injektion enthält, eine distale Spitze (23), die mit einer Injektionsnadel (22) versehen ist, und einen Stopfen (25) in Verschiebungseingriff mit dem Zylinder umfasst, wobei der medizinische Behälter in dem Körper (10) zwischen einer proximalen Position und einer distalen Position verschiebbar angeordnet ist;
- eine Sicherheitsabschirmung (60), die in dem Körper (10) zwischen einer distalen anfänglichen Position und einer proximalen zweiten Position verschiebbar gelagert ist;
- eine Kolbenstange (40), die verschiebbar in dem Körper (10) angeordnet ist und einen hohlen Schaft (41) umfasst, der einen inneren axialen Anschlag (45) und einen distalen Flansch (42) umfasst, der dazu geeignet ist, den Stopfen (25) in der distalen Richtung zu drücken, wobei die Kolbenstange einen proximalen Flansch (43) umfasst, der mittels mindestens eines biegsamen Arms (140) des Körpers lösbar in der anfänglichen Position verriegelt ist;
- einen Nocken (70), welcher derart mit der Sicherheitsabschirmung (60) zusammenwirkt, dass eine Translation der Sicherheitsabschirmung (60) von der distalen anfänglichen Position zur proximalen zweiten Position den Nocken (70) veranlasst, sich innerhalb des Körpers (10) zu einer Entriegelungsposition zu bewegen;
- ein Aktivierungsorgan (80), das verschiebbar an einem proximalen Ende des Körpers (10) montiert ist und mindestens ein Bein (83) umfasst, das sich in der distalen Richtung erstreckt, wobei jedes Bein (83) einen Zahn (82) umfasst, der derart in einer dritten Nut (73) des Nockens (70) aufgenommen ist, dass die Drehung des Nockens (70) zu einer Entriegelungsposition das Aktivierungsorgan (80) veranlasst, sich distal translatorisch zu bewegen, um die biegsamen Arme (140) nach außen auszulenken, um den proximalen Flansch (43) der Kolbenstange freizugeben, wobei das Aktivierungsorgan (80) zwischen einer anfänglichen Position, in der das Aktivierungsorgan proximal von den biegsamen Armen (140) beabstandet ist, und einer Entriegelungsposition beweglich ist, in der das Aktivierungsorgan die biegsamen Arme (140) veranlasst, nach außen ausgelenkt zu werden, um den proximalen Flansch (43) der Kolbenstange freizugeben, wodurch es der Kolbenstange erlaubt wird, sich in der distalen Richtung zu bewegen; und
- ein mittleres Teil (90), das einen proximalen Kopf (91, 93) und einen distalen Stopp (92) umfasst, wobei das mittlere Teil (90) sich derart in dem hohlen Schaft (41) der Kolbenstange erstreckt, dass der proximale Kopf mittels eines Rückhalteelements (85) des Aktivierungsorgans proximal von dem proximalen Flansch (43) der Kolbenstange in der anfänglichen Position verriegelt wird, derart dass, wenn die Kolbenstange sich in der distalen Richtung bewegt, die Kolbenstange distal entlang des mittleren Teils (90) verschoben wird, bis der Stopp (92) mit dem Anschlag (45) ineinandergreift,
wobei das Rückhalteelement (85), der Stopp (92), der proximale Kopf (91, 93) und der Anschlag (45) dazu ausgestaltet sind, das Außer-Eingriff-Bringen entweder des mittleren Teils (90) aus dem Aktivierungsorgan (80) oder der Kolbenstange (40) aus dem mittleren Teil (90) am Ende der Injektion zu erlauben, derart dass das Außer-Eingriff-Bringen die Erzeugung eines Tons ergibt.

2. Automatische Injektionsvorrichtung nach Anspruch 1, wobei das Rückhalteelement (85) des Aktivators mindestens einen Vorsprung umfasst, der sich radial nach innen erstreckt, wobei der Vorsprung mit dem proximalen Ende (91, 93) ineinandergreift, derart dass zumindest bis zum Ende der Injektion verhindert wird, dass das mittlere Teil (90) sich in der distalen Richtung bewegt.

3. Automatische Injektionsvorrichtung nach Anspruch 2, wobei der proximale Kopf (91) des mittleren Teils eine Gabelform mit zwei proximalen Beinen aufweist und der proximale Kopf dazu ausgestaltet ist, am Ende der Injektion aus dem Vorsprung (85) des Aktivierungsorgans außer Eingriff zu gelangen, wodurch ein Ton erzeugt wird.

4. Automatische Injektionsvorrichtung nach Anspruch 2, wobei der proximale Kopf (91) des mittleren Teils eine Gabelform mit zwei proximalen Beinen aufweist und der Vorsprung des Aktivierungsorgans dazu ausgestaltet ist, das mittlere Teil (90) bis nach der Injektion zurückzuhalten, und die Kolbenstange (40) dazu ausgestaltet ist, am Ende der Injektion aus dem distalen Stopp (92) des mittleren Teils (90) außer Eingriff gebracht zu werden, wodurch ein Ton erzeugt wird.

5. Automatische Injektionsvorrichtung nach Anspruch 2, wobei der proximale Kopf (93) des mittleren Teils eine Harpunenform mit zwei distalen Haken aufweist, der proximale Kopf sich in einem freien Zustand in der anfänglichen Position befindet und der proximale Kopf dazu ausgestaltet ist, am Ende der Injektion durch Zusammendrücken der Haken radial nach innen und dann Lösen der Zusammendrückungen in den Haken von dem Vorsprung (85) des Aktivierungsorgans außer Eingriff gebracht zu werden, wodurch ein Ton erzeugt wird.

6. Automatische Injektionsvorrichtung nach einem der Ansprüche 1 bis 5, wobei jeder biegsame Arm (140) eine geneigte innere Oberfläche (16), die nach innen in der distalen Richtung (16) verjüngt ist, und mindestens einen Kolbenhalter (17) umfasst, der distal von der geneigten Oberfläche (16) angeordnet ist, wobei der proximale Flansch (43) der Kolbenstange in der anfänglichen Position mit dem mindestens einen Halter (17) in Anschlag steht.

7. Automatische Injektionsvorrichtung nach Anspruch 6, wobei das Aktivierungsorgan (80) mindestens einen inneren Vorsprung (84) umfasst, der sich in der distalen Richtung erstreckt, wobei der mindestens eine Vorsprung (84) in der anfänglichen Position proximal von der geneigten Oberfläche (16) eines entsprechenden biegsamen Arms (140) beabstandet ist und wobei, wenn der Nocken (70) sich in der Entriegelungsposition befindet, der mindestens eine innere Vorsprung mit der entsprechenden geneigten Oberfläche (16) ineinandergreift, um den biegsamen Arm (140) nach außen auszulenken.

8. Automatische Injektionsvorrichtung nach einem der Ansprüche 1 bis 7, wobei die Sicherheitsabschirmung (60) mindestens einen Ansatz (62) umfasst, der derart in einer entsprechenden ersten Nut (71) des Nockens (70) aufgenommen ist, dass eine Bewegung der Sicherheitsabschirmung (60) in der proximalen Richtung den Nocken veranlasst, sich zu drehen.

9. Automatische Injektionsvorrichtung nach Anspruch 8, wobei die erste Nut (71) einen geneigten ersten Zweig (71a) und einen axialen zweiten Zweig (71b) aufweist, der mit dem ersten Zweig am proximalen Ende verbunden ist, derart dass in der anfänglichen Position der Ansatz (62) sich am distalen Ende des ersten Zweigs (71a) befindet und sich hin zum proximalen Ende des ersten Zweigs bewegt, wenn die Sicherheitsabschirmung (60) sich von der anfänglichen Position zur zweiten Position bewegt.

10. Automatische Injektionsvorrichtung nach einem der Ansprüche 1 bis 9, wobei die Kolbenstange (40) in der distalen Richtung von einer ersten Feder (49) gedrängt wird.

11. Automatische Injektionsvorrichtung nach einem der Ansprüche 1 bis 10, wobei die Sicherheitsabschirmung (60) in der distalen Richtung von einer zweiten Feder (65) gedrängt wird.

12. Automatische Injektionsvorrichtung nach einem der Ansprüche 1 bis 11, ferner umfassend einen Ring (50), der den medizinischen Behälter (20) stützt, wobei der Ring mindestens einen Finger (53) umfasst, der radial nach außen hervorsteht, der Nocken (70) ferner mindestens eine zweite Nut (72) umfasst, die den jeweiligen mindestens einen Finger (53) aufnimmt, wobei die mindestens eine zweite Nut (72) einen proximalen Abschnitt (72a), einen distalen Abschnitt (72c) und einen geneigten Abschnitt (72b) umfasst, der den proximalen Abschnitt (72a) mit dem distalen Abschnitt (72c) verbindet, wobei der Ring (50) an dem proximalen Abschnitt (72a) der zweiten Nut (72) verriegelt ist und sich entlang des geneigten Abschnitts (72c) zum distalen Abschnitt (72b) erstreckt, wenn die Kolbenstange den medizinischen Behälter von der anfänglichen Position zur Injektionsposition drückt.

13. Automatische Injektionsvorrichtung nach Anspruch 12, wobei der mindestens eine Finger (53) mit einer axialen Nut (15) des Körpers ineinandergreift.

## Revendications

1. Dispositif d'injection automatique (1) comprenant :
- un corps (10) ;
- un récipient médical (20) comprenant un cylindre (24) contenant un produit à injecter, une pointe distale (23) munie d'une aiguille d'injection (22) et un bouchon (25) en engagement coulissant à l'intérieur du cylindre, le récipient médical étant disposé de manière coulissante dans le corps (10) entre une position proximale et une position distale ;
- un bouclier de sécurité (60) monté de manière coulissante à l'intérieur du corps (10) entre une position initiale distale et une deuxième position proximale ;
- une tige de piston (40) disposée de manière coulissante dans le corps (10), comprenant un arbre creux (41) comportant une butée axiale interne (45) et une bride distale (42) adaptée pour pousser le bouchon (25) dans la direction distale, la tige de piston comprenant une bride proximale (43) verrouillée de manière libérable dans la position initiale par au moins un bras flexible (140) du corps ;
- une came (70) coopérant avec le bouclier de sécurité (60) de telle sorte qu'une translation du bouclier de sécurité (60) de la position initiale distale à la deuxième position proximale provoque la rotation de la came (70) à l'intérieur du corps (10) vers une position de déverrouillage ;
- un activateur (80) monté de manière coulissante à une extrémité proximale du corps (10) et comprenant au moins une jambe (83) s'étendant dans la direction distale, chaque jambe (83) comprenant une dent (82) reçue dans une troisième rainure (73) de la came (70), de sorte que la rotation de la came (70) vers une position de déverrouillage provoque la translation de l'activateur (80) vers l'extrémité distale afin de dévier les bras flexibles (140) vers l'extérieur pour libérer la bride proximale (43) de la tige de piston, l'activateur (80) étant mobile entre une position initiale dans laquelle l'activateur est espacé de manière proximale des bras flexibles (140) et une position de déverrouillage dans laquelle l'activateur provoque la déviation vers l'extérieur des bras flexibles (140) afin de libérer la bride proximale (43) de la tige de piston, permettant ainsi à la tige de piston de se déplacer dans la direction distale ; et
- une pièce centrale (90) comprenant une tête proximale (91, 93) et une butée d'arrêt distale (92), la pièce centrale (90) s'étendant dans l'arbre creux (41) de la tige de piston de telle sorte que la tête proximale est verrouillée par un élément de retenue (85) de l'activateur à proximité de la bride proximale (43) de la tige de piston dans la position initiale, de sorte que, lorsque la tige de piston se déplace dans la direction distale, la tige de piston coulisse distalement le long de la pièce centrale (90) jusqu'à ce que la butée d'arrêt (92) s'engage contre la butée (45),
dans lequel l'élément de retenue (85), la butée d'arrêt (92), la tête proximale (91, 93) et la butée (45) sont conçus pour permettre de désengager soit la pièce centrale (90) de l'activateur (80), soit la tige de piston (40) de la pièce centrale (90) à la fin de l'injection, de telle sorte que ledit désengagement entraîne la génération d'un son.

2. Dispositif d'injection automatique selon la revendication 1, dans lequel l'élément de retenue (85) de l'activateur comprend au moins une saillie s'étendant radialement vers l'intérieur, ladite saillie s'engageant dans la tête proximale (91, 93) de manière à empêcher la pièce centrale (90) de se déplacer dans la direction distale au moins jusqu'à la fin de l'injection.

3. Dispositif d'injection automatique selon la revendication 2, dans lequel la tête proximale (91) de la pièce centrale a une forme de fourche avec deux jambes proximales et la tête proximale est configurée pour se désengager de la saillie (85) de l'activateur à la fin de l'injection, générant ainsi un son.

4. Dispositif d'injection automatique selon la revendication 2, dans lequel la tête proximale (91) de la pièce centrale a une forme de fourche avec deux jambes proximales et la saillie de l'activateur est configurée pour retenir la pièce centrale (90) jusqu'après l'injection et la tige de piston (40) est configurée pour se désengager de la butée d'arrêt distale (92) de la pièce centrale (90) à la fin de l'injection, générant ainsi un son.

5. Dispositif d'injection automatique selon la revendication 2, dans lequel la tête proximale (93) de la pièce centrale a une forme de harpon avec deux crochets distaux, la tête proximale étant dans un état libre dans la position initiale et la tête proximale étant configurée pour se désengager de la saillie (85) de l'activateur à la fin de l'injection en contraignant les crochets radialement vers l'intérieur puis en relâchant les contraintes dans les crochets, générant ainsi un son.

6. Dispositif d'injection automatique selon l'une quelconque des revendications 1 à 5, dans lequel chaque bras flexible (140) comprend une surface intérieure inclinée (16) s'effilant vers l'intérieur dans la direction distale et au moins un dispositif de retenue du piston (17) disposé distalement par rapport à ladite surface inclinée (16), la bride proximale (43) de la tige de piston étant en butée avec l'au moins un dispositif de retenue (17) dans la position initiale.

7. Dispositif d'injection automatique selon la revendication 6, dans lequel l'activateur (80) comprend au moins une saillie interne (84) s'étendant dans la direction distale, ladite au moins une saillie (84) étant espacée de manière proximale de la surface inclinée (16) d'un bras flexible respectif (140) dans la position initiale et dans lequel, lorsque la came (70) est dans la position de déverrouillage, ladite au moins une saillie interne s'engage dans la surface inclinée respective (16) pour fléchir le bras flexible (140) vers l'extérieur.

8. Dispositif d'injection automatique selon l'une quelconque des revendications 1 à 7, dans lequel le bouclier de sécurité (60) comprend au moins une patte (62) reçue dans une première rainure respective (71) de la came (70) de sorte qu'un mouvement du bouclier de sécurité (60) dans la direction proximale provoque la rotation de la came.

9. Dispositif d'injection automatique selon la revendication 8, dans lequel la première rainure (71) présente une première branche inclinée (71a) et une deuxième branche axiale (71b) reliée à la première branche à une extrémité proximale, de telle sorte que, dans la position initiale, l'ergot (62) est situé à l'extrémité distale de la première branche (71a) et se déplace vers l'extrémité proximale de la première branche lorsque le bouclier de sécurité (60) se déplace de la position initiale à la deuxième position.

10. Dispositif d'injection automatique selon l'une quelconque des revendications 1 à 9, dans lequel la tige de piston (40) est poussée dans la direction distale par un premier ressort (49).

11. Dispositif d'injection automatique selon l'une quelconque des revendications 1 à 10, dans lequel le bouclier de sécurité (60) est poussé dans la direction distale par un deuxième ressort (65).

12. Dispositif d'injection automatique selon l'une quelconque des revendications 1 à 11, comprenant en outre une bague (50) supportant le récipient médical (20), la bague comprenant au moins un doigt (53) faisant saillie radialement vers l'extérieur, la came (70) comprenant en outre au moins une deuxième rainure (72) recevant l'au moins un doigt (53) respectif, l'au moins une deuxième rainure (72) comprenant une partie proximale (72a), une partie distale (72c) et une partie inclinée (72b) reliant la partie proximale (72a) à la partie distale (72c), dans lequel la bague (50) est verrouillée au niveau de la partie proximale (72a) de la deuxième rainure (72) et se déplace le long de la partie inclinée (72c) vers la partie distale (72b) lorsque la tige de piston pousse le récipient médical de la position initiale à la position d'injection .

13. Dispositif d'injection automatique selon la revendication 12, dans lequel l'au moins un doigt (53) s'engage dans une rainure axiale (15) du corps.
